# EUROPEAN PATENT APPLICATION

(11) **EP 3 147 031 A1**
(43) Date of publication of application: **29.03.2017**
(21) Application number: 15186753.8
(22) Date of filing: 24.09.2015
(51) Int. Cl.: B05B 7/24, B05B 15/00, B05B 7/00, A61L 9/14

(54) **FLUID DISPERSAL SYSTEM**

(71) Applicant: IDFC AG, 9494 Schaan (LI)
(72) Inventor: ROBERTS, Stephen Mark, Troon, KA 10 6TA (GB); BATRAM, Mathias Joerg, D-82031 Gruenwald (DE)
(74) Representative: Kaminski Harmann

(57) **Abstract**

The invention pertains to a fluid dispersal system (100) comprising a diffuser means (110) with a diffusion chamber (140) adapted to disperse a fluid (50) by means of cold air diffusion, wherein the diffuser means (110) comprises an air intake (130) having an air intake orifice through which compressed air is introducible into the diffusion chamber (140), a fluid import (155) having a fluid import orifice through which the fluid (50) is introducible into the diffusion chamber (140), a mix exhaust port (145) through which an air and fluid mix from the diffusion chamber (140) is introducible into a mix chamber, and a mist output vent (170) that is connected to the mix chamber and adapted to allow a dispersion from the mix chamber to be dispersed out of the system (100), characterized in that a ratio of cross-sectional areas of the air intake orifice to the fluid import orifice is at least 5:1. The invention furthermore pertains to a fluid container (200) for use with the fluid dispersal system and to a method for dispersing a fluid (50) by means of cold air diffusion.

## Description

The present invention pertains to a fluid dispersal system operating with cold air diffusion and particularly having an exchangeable fluid container, to an exchangeable fluid container for use with the fluid dispersal system, and to a method for dispersing a fluid by means of cold air diffusion.

Cold air diffusion is a known method to disperse fragrance oil into the air, it combines a diffuser and an air compressor and it is designed through the dimensions and the relationships that various ports and orifices have to each other, to minimise the amount of fluid oil that is converted into scented mist - in order to make the fluid last as long as possible.

These devices have a tube which typically is connected in an off-set asymmetrical manner to the underside of the diffuser, and this extends into the oil reservoir which is screw-fixed to the underside of the device. As essential fragrance oils are aggressive chemicals and they coat the tube with a residue and can easily contaminate a user or burn the eyes, it requires skilled staff to exchange reservoirs when they require to be replaced.

Current cold air diffusion devices typically operate under a pressure in the region of 2.7 bar (39 psi) and they force air through two orifices with the first being larger than the second. This speeds the air up and the resulting mist which is created often contains large wet particles which reduces the effect of the device and requires a drying tower. Such a device is shown e. g. in WO 2005/105163 A1. Typically, approximately 1 to 2 ml oil will be used per hour with such a device.

A current doctrine when seeking to increase the amount of scented mist that is output into an area is to add additional machines. This is however costly. It is therefore an object of the invention to provide an improved diffusion head be capable of outputting significantly higher levels of scent mist from a single unit.

It is another object to ensure only authentic refill cartridges can be used and furthermore that empty units that have been refilled unofficially will also not function with the device.

It is a further object of the present invention to allow an easy connection of the replaceable fluid containers to the diffusion device and to reduce the risk of contamination when replacing them, as this allows unskilled staff to replace empty containers without reliance on expensive contractors or specially trained service personnel.

At least one of these objects is achieved by the fluid dispersal system according to claim 1, the fluid container according to claim 13, the method according to claim 14 and/or the dependent claims of the present invention.

Applicant has discovered that a significant performance improvement can be achieved by altering the ratios between certain of the orifices or by reducing the supply pressure from the compressor. This discovery allows for a system to output more scent fragrance while operating under lower pressure for less time which makes it suited to DC battery power - which is desirable in many locations where an AC power supply is difficult / expensive to provide.

Applicant has further discovered that the ratios between the orifices are critical to the performance of such devices and their ability to output dry particulate matter, and that by reversing current thinking on orifice size and altering the ratios in combination with reducing the air pressure by 90%, a significant performance improvement in the range of 200% to 800% can be achieved. By introducing multiple cells it becomes possible to increase output to levels of mist capable of delivering not just high levels of fragrance scent but also efficacy for pest and pathogen control.

In one embodiment of the system the feeding tube that allows the chemical to be drawn up into the device does not extend from the diffuser and is in fact an integral element of the oil reservoir.

In another embodiment of the system, a supply orifice located within the diffuser (and which would have been connected to the tube) is repositioned in a central position on the underside of the diffuser to allow it to connect via a small male probe to the tube which is now integrally part of the reservoir. This repositioning requires a re-design of diffusion heads which currently feature an air pressure jet / mixing orifice in the central position. By re-positioning the feeder tube centrally it allows for easy connection with the integral feeder tube within the reservoir when the two elements are screw fit together. (This connection would not be possible in the current arrangement as it is impossible to determine where any off-set feature would be in relation to any element integrated within a reservoir that required being screw fit to achieve a seal). These parts may also be compression or bayonet fitted.

In one embodiment, RFID or similar technology are used to ensure only authentic refill cartridges can be used and furthermore that empty units that have been refilled unofficially will also not function with the device. It is intended that the device will read the ID tag of refills and that it will not accept any refill previously "mated" to the device, as it's on board control circuitry will be able to reference its usage data to detect such an action and prevent operation in such an eventuality. Other identification methods such as barcodes or QR codes may also be used as alternatives to RFID.

An fluid dispersal system according to the invention comprises a diffuser means with a diffusion chamber adapted to disperse a fluid by means of cold air diffusion. The diffuser means comprises
- an air intake having an air intake orifice through which compressed air is introducible into the diffusion chamber,
- a fluid import having a fluid import orifice through which the fluid is introducible into the diffusion chamber,
- a mix exhaust port through which an air and fluid mix from the diffusion chamber is introducible into a mix chamber, and
- a mist output vent that is connected to the mix chamber and adapted to allow a dispersion from the mix chamber to be dispersed out of the system.

According to the invention, a ratio of cross-sectional areas of the air intake orifice to the fluid import orifice is at least 5:1.

In one embodiment, the ratio of cross-sectional areas of the air intake orifice to the fluid import orifice is at least 30:1, in particular at least 59:1.

In another embodiment, the air intake comprises an air jet nozzle having a jet nozzle orifice, and the ratio of cross-sectional areas of the jet nozzle orifice to the air intake orifice is at least 0.5:1, particularly at least 0.99:1.

In a further embodiment, the mist output vent has an output orifice, and the ratio of cross-sectional areas of the output orifice to the fluid import orifice is at least 10:1, particularly at least 99:1.

In a further embodiment of a fluid dispersal system according to the invention, the fluid import comprises a Venturi tube, and the diffuser means is adapted to draw the fluid through the fluid import into the diffusion chamber by means of the Venturi effect.

In one embodiment, in the diffusion chamber the fluid import is positioned oppositely the exhaust port.

In another embodiment, the fluid import has an annular orifice. The first feeder tube coupling is then preferably positioned in a centre of the annular orifice.

In a further embodiment, the fluid import comprises a multitude of fluid import units, i.e. at least three. Preferably also a corresponding number of exhaust port units is provided and in the diffusion chamber each of the multitude of exhaust port units is positioned oppositely a fluid import unit.

In one embodiment, the output vent is adapted to introduce the dispersion to a fan or an air conditioning or ventilation system from which the dispersion is dispersed.

In a special embodiment, the system comprises a fluid container for providing the fluid. In one embodiment, the system comprises a common housing for the diffuser means and the fluid container. Particularly, the system further comprises
- a timer and programming module adapted to control a dispersal programme of the diffuser means,
- at least one air compressor, and/or
- an accumulator battery and means for recharging the accumulator battery, particularly comprising photovoltaic cells or means for connecting a power supply cord.

In another special embodiment, the system comprises an exchangeable fluid container for providing the fluid. In one embodiment, the diffuser means comprises accommodation means adapted to accept the fluid container, and a feeder tube extension with a first feeder tube coupling. Further, the diffuser means is adapted to connect the fluid container to the fluid import so that the first feeder tube coupling couples with a second feeder tube coupling of a feeder tube of the fluid container, and to draw the fluid through the feeder tube and the feeder tube extension.

In another embodiment, the first and second feeder tube couplings are adapted as a male probe and a female receiver and to create a fluid tight connection between the feeder tube extension and the feeder tube.

In yet another embodiment, the accommodation means is adapted to connect the fluid container to the diffuser means so that the mix chamber is created at least partially inside the fluid container.

In one embodiment, the accommodation means comprises a screw thread and is adapted to accept the fluid container by means of screwing, and the feeder tube coupling is arranged at a centre point of the screw thread.

In another embodiment, the accommodation means is adapted to accept a plurality of exchangeable fluid containers simultaneously, comprises a plurality of feeder tube extensions, and is adapted to connect the plurality of fluid containers to the diffuser means so that each of the feeder tube extensions couples with a feeder tube of one of the plurality of fluid containers.

In a further embodiment, the system comprises sensor means for reading a machine-readable code provided on the fluid container and for identifying, based on the code, the fluid container and/or a fluid contained by the fluid container, particularly wherein the sensor means comprises a camera, a barcode scanner or an RFID reader.

The first aspect of the invention also relates to an exchangeable fluid container that is adapted to contain a fluid and for providing the fluid to a diffuser means of a fluid dispersal system according to this aspect of the invention when being connected to the diffuser means. The fluid container comprises a feeder tube with a second feeder tube coupling and is adapted to be connected to the diffuser means in such a way that the second feeder tube coupling couples with the first feeder tube coupling of the diffuser means.

The first aspect of the invention also relates to a method for dispersing a fluid by means of cold air diffusion. The method comprises introducing air with an air intake pressure through an air intake into a diffusion chamber, and introducing the fluid with a fluid import pressure through a fluid import into the diffusion chamber. According to the invention, the fluid import pressure is significantly higher than the air intake pressure.

In a particular embodiment, the method is performed with a fluid dispersal system according to the first aspect of the invention.

The fluid import pressure preferably is at least 50% higher than the air intake pressure, particularly at least 100% higher or at least five times as high.

In another embodiment, the method comprises connecting a fluid container to a diffuser means, wherein the fluid container provides the fluid, the diffuser means comprises the diffusion chamber, and connecting comprises coupling a feeder tube of the fluid container to a feeder tube extension of the diffuser means.

In a further embodiment, the method according to the first aspect of the invention comprises drawing the fluid through the fluid import by means of the Venturi effect, the air intake pressure e. g. being less than 0.3 bar.

### LIQUID LEVEL INDICATOR SYSTEM

A second aspect of the present invention relates to a liquid level indicator system for a fluid container.

It is desirable to be able to determine the fluid level in refill cartridges (e. g. for a refillable fluid dispersal system according to the first aspect of the invention).

It is furthermore desirable to provide an alert if a fluid level drops so that the refill cartridge needs to be replaced.

It is therefore an object of this aspect of the present invention to provide a liquid level indicator system that allows automatic detection of a fluid level in a fluid container.

It is a further object to provide such a liquid level indicator system for a refill cartridge of a refillable fluid dispersal system.

Another object of this aspect of the present invention is to provide a fluid container for use with such a liquid level indicator system.

A liquid level indicator system according to this aspect of the invention for determining a level of a fluid comprises a sensor unit with at least one electronic sensor means and a fluid container comprising a body with a corpus part enclosing a volume for accommodating the fluid.

According to a first design of the system, a floater is provided inside of the volume, wherein the floater is adapted to float on the fluid and comprises a transponder, particularly an RFID transponder, that is identifiable by the electronic sensor means. The sensor unit is adapted to deduce a level of the fluid in the volume based on an identification of the transponder by at least one of the electronic sensor means.

In a particular embodiment of the first design, the liquid level indicator system is part of a fluid dispersal system according to the first or third aspect of the invention, particularly wherein the floater is provided at the feeder tube of the fluid container.

In another particular embodiment of the first design, the fluid container is removable from the sensor unit. It is positionable with respect to the sensor unit in such a way that either
- at least one electronic sensor means is provided below the fluid container, wherein identification of the transponder is interpreted by the sensor unit as a relatively low level; and/or
- at least one electronic sensor means is provided above the fluid container, wherein identification of the transponder is interpreted by the sensor unit as a relatively high level.

In one embodiment, the at least one electronic sensor mean is adapted to identify the transponder through the body of the fluid container only at a range of below two centimetres, in particular below five millimetres.

Very low power RFID or similar technology can be used to facilitate this desired functionality where the transponder will float on top of the fluid within the refill, only becoming "detectable" if it gets extremely close to the detection element situated under the refill. Alternatively the same method may be used in reverse, whereby the RFID transponder is detectable from above as it floats on the surface and that as it gets lower in the reservoir and becomes undetectable that this triggers the alert to replace the empty reservoir.

It is also possible to use capacitance probes punctured through the side of the reservoir at low level and connected to the processor on board the device to detect when it is time to replace an empty fluid reservoir:
According to a second design of the system, the fluid is conductive and the electronic sensor means is adapted as capacitance probe of the fluid container. The capacitance probe comprises an upper probe and a lower probe which are positioned inside of the fluid container in such a way that an electrical connection is established between the upper probe and the lower probe if the level of the fluid lies above the upper probe, and that no electrical connection is established if the level of the fluid lies below the upper probe.

An alarm can be issued to either the local operator or indeed the supplier of the technology when a refill is determined to be almost empty.

In one embodiment of the first or second design, output means are provided which are adapted to signalize the deduced level
- to a user, particularly by means of light signals,
- to a device or system, the liquid level indicator system is a part of or attached to; and/or
- to a provider of the fluid container, particularly via an Internet connection.

A fluid container comprises a body with a corpus part enclosing a volume for accommodating a fluid. According to this aspect of the invention, a floater is provided inside of the volume of the fluid container, wherein the floater is adapted to float on the fluid and comprises a transponder, e. g. an RFID transponder. The transponder and the body of the fluid container are adapted in such a way that the transponder is identifiable by electronic sensor means if these are positioned outside the fluid container, allowing deducing a level of the fluid in the volume based on an identification of the transponder by the electronic sensor means.

In one embodiment, the fluid container according to this aspect of the invention comprises a neck part for connecting the container to an external device in order to provide the fluid to this device, and a feeder tube that extends from the neck into the volume, wherein the floater is provided at the feeder tube of the fluid container.

In one embodiment, the external device comprises a liquid level indicator system according to the first design of this aspect of the invention, particularly wherein the external device is a fluid dispersal system according to the first or third aspect of the invention.

In another embodiment, the neck part comprises a screw thread and a centre point of a circular diameter of the feeder tube correlates with a centre point of a circular diameter of the screw thread.

### SCREWING CONNECTION

It is known that fragrance, scents and other fluids can be dispersed into the air economically and over considerable distance by cold air diffusion: Pressurised air is passed through a specially designed chamber in which it mixes with essential oils or other fluids to create a fine vapour which is then dispersed into the surrounding air. Such devices currently require mating with a container or reservoir (refill) which contains the fluid. They are equipped with a "feeder tube" which is connected to and extends from the cold air diffusion device (diffuser head) and is submerged into the fluid within the refill. The feeder tube allows the fluid to be drawn up into the mixing chamber within the cold air diffusion device, where it is vaporised and dispersed via air convection or via introduction to an air conditioning or ventilation system or a fan.

A number of problems are associated with known fragrance dispersal devices and methods:
A first problem relates to possible contamination with the fragrance fluid comprising essential oils. Such contamination with essential oils is to be avoided, as in concentrated form they are very aggressive, they aggravate and can burn the skin; they leave an extremely strong smell and can permanently stain materials. Although current diffuser heads vary in design, they all require a feeder tube to be attached at the underside of the device. This feeder tube makes it difficult for a user of such a fluid dispersal system to change from one used refill to another new refill without contaminating the hands, clothes or surrounding environment, as the residual essential oil coats the outside of the feeding tube and is also contained inside the tube (even when the refill is empty) and, thus, often touches the hands, drips out of the tube or - especially if the feeder tube is flexible - is transferred via "splatter" onto other surfaces in the immediate area, as a flexible tube is prone to spring back and forth spraying oil if accidentally touched. These fragrance systems are often deployed in areas where staff have limited technical knowledge and lack the training required to allow them to replace fragrance oil safely and without contaminating themselves or their clothing. This means that in many cases, disadvantageously, the re-supply has to be carried out by external parties at a significant cost.
A second problem relates to the sealing of the fluid containers. As the known methods of providing essential oil and mating it to a cold air diffuser are by way of a refill vessel made from glass, plastic or metal which has a screw thread allowing it to be screwed into the bottom of the diffuser, effecting an air tight and pressure capable seal and such refill requires to have an open top to allow the fragrance mist to rise into the diffuser, it is impractical to have them sealed, as this would render them useless. This makes it impossible to ensure the integrity and quality of the fluid as there is no hermetic seal, often causes spillage during transit as screw top lids and their temporary seals are not suited to the function and are often damaged or breached by the oil contained inside.

Furthermore, the lack of a sealed unit makes it very easy to refill an old fluid container refill with unapproved oil. This can affect the performance of the system, has considerable environmental risks and undermines supplier revenue if the oil is not purchased from the official supplier.

The document WO 2008/098597 A1 describes a device for releasing fragrances for use with exchangeable pressure containers comprising the fragrance, wherein for identification of the pressure containers, the device and the exchangeable pressure containers each comprise a data processing unit with a non-volatile random access memory.

However, for systems having an exchangeable liquid container, particularly being adapted for cold air diffusion, a method for preventing unauthorised use of non-original containers or for preventing unauthorised refilling of original containers would be desirable.

It is therefore an object of this aspect of the present invention to provide an improved fluid dispersal system that is suited for dispensing vapour from fluids provided by exchangeable fluid container, wherein the change of the container is facilitated and less risky with respect to contamination.

It is a further object to provide such a system that is safely operable by unskilled users.

It is a further object to provide such a system that prevents the use of non-original containers or the unauthorised use of refilled containers with the system.

It is a particular object of the invention to provide this prevention with a less complex and expensive technique, particular wherein the exchangeable containers comprise less complex and expensive safety features.

It is another object of the invention to provide an improved fluid container for use with this system.

At least one of these objects is achieved by a fluid dispersal system, the liquid container and the insert as described below.

The system according to this aspect of the invention comprises the following features:
The diffuser head is designed in such a way that it does not require a feeder tube, as this essential component is incorporated within a refill. The diffuser head has an arrangement making it physically possible to achieve the required pressure between fittings and therefore for cold air diffusion to occur when it is mated to a self-contained refill device comprising the feeder tube.

Optionally, the diffuser head may be designed in such a way that when the sealed refill is screwed, compressed or otherwise mounted to the diffuser head, there is a tooth arrangement on the bottom of the diffuser head which pierces and slices off the seal membrane as it is offered up to the diffuser head, allowing cold air diffusion to occur.

The part of the diffuser head which provides the mating capability to ensure the required pressure is achieved between the diffuser and the feeder tube which is enclosed and incorporated within the refill and which optionally also removes the membrane seal. This is attached to the bottom of the diffuser by way of a screw threaded aluminium or other non-specified metal cylindrical jacket. This jacket is capable of being fitted to other commercially available diffuser heads and thus could be retro-fitted to any other diffuser system.

An aluminium, plastic or glass fluid container has an additional part added which is described as a plastic or metal component insert, which is inserted within the neck of the container. This insert comprises the feeder tube and the mating ports required to connect the device to the diffuser head thus allowing cold air diffusion to occur in this new arrangement without impeding a pressure seal being achieved between the refill and the threaded screw or compression fitting arrangements that allow the diffuser head and refill to mate with each other.

Preferably, the insert has a hermetically attached and heat sealed plastic or aluminium membrane on its uppermost side which prevents spillage or escape of concentrated essential oil or scent smell until such times as the device has been fully inserted and mated to the cold air diffuser. The membrane can be removed prior to inserting the container into the device. If there are teeth provided on the underside of the diffuser head for removing the seal, the insert is preferably designed and positioned such that the membrane can be disrupted and removed without the teeth damaging the actual insert.

In addition to original fluid containers being sealed to offer some protection against unapproved refill or oil supply, optionally a barcode identification miniature camera system is incorporated within the inner case of the fragrance unit. This scans a unique UV activated barcode which is invisible to the human eye. This detection and identification system interfaces with the device's control timer printed circuit board and processor system and it will not permit the machine to operate unless it detects an original code that is within pre-programmed parameters nor will it accept any code that it has previously recognised and approved. Reading out the code allows determining the correct amount of fluid to be dispensed, and also helps to prevent that someone accidentally or intentionally applies unapproved fluids that might cause damage to the device as well as to the health of the users.

A refillable fluid dispersal system comprises a diffuser means adapted to disperse a fluid into the air, and an exchangeable fluid container that is mountable to the diffuser means for providing the fluid to the diffuser means. The system further comprises accommodation means that are adapted for accepting the fluid container and connecting the fluid container to the diffuser means.

According to this aspect of the invention, the exchangeable fluid container comprises a neck part, a feeder tube which extends from the neck part into a volume of the fluid container that is adapted to contain the fluid, and a seal that is adapted to prevent fluid from exiting the fluid container before the fluid container is mounted to the accommodation means. The accommodation means is adapted to accept the neck part of the fluid container so that the fluid container is mountable to the accommodation means, and a feeder tube coupling that is adapted to couple with the feeder tube when the fluid container is mounted to the accommodation means.

In one embodiment, the neck part has a first screw thread, and the accommodation means have a second screw thread that is adapted to accept the first screw thread of the fluid container so that the fluid container is mountable to the accommodation means by means of screwing.

In another embodiment, the neck part and the accommodation means together form a compression fitting for mounting the container to the accommodation means.

In yet another embodiment, the neck part and the accommodation means together form a bayonet mount for mounting the container to the accommodation means.

In one embodiment of the refillable fluid dispersal system, the diffuser means comprises a cold air diffusion chamber and is adapted to draw the fluid through the feeder tube and the feeder tube coupling into the diffusion chamber, to aerolize or vaporize the fluid, and to disperse the aerolized or vaporized fluid into the air, particularly by means of air convection or by introduction to an air conditioning or ventilation system or a fan.

In another embodiment the refillable fluid dispersal system, comprises a diffuser means with a cold air diffusion chamber adapted to vaporize the fluid, wherein the diffuser means comprises
- an air intake through which compressed air is drawn into the cold air diffusion chamber,
- a feeder tube extension with a fluid import through which the fluid is drawn into the cold air diffusion chamber,
- a mix exhaust port through which an air and fluid mix from the cold air diffusion chamber is drawn into a mix chamber, and
- a mist output vent that is connected to the mix chamber and adapted to allow vapour from the mix chamber to be dispersed out of the system.

In one embodiment the fluid import comprises a Venturi tube, and the diffuser means is adapted to draw the fluid through the feeder tube, the feeder tube extension and the fluid import into the cold air diffusion chamber by means of the Venturi effect.

In another embodiment of the fluid dispersal system according to this aspect of the invention, the accommodation means comprises a bottom face that is adapted to contact a top face of the neck part of the fluid container when the fluid container is mounted to the accommodation means.

In a further embodiment of the fluid dispersal system, the accommodation means and the neck part are adapted to create a liquid-tight connection when the fluid container is mounted, e. g. screwed, to the accommodation means.

In yet another embodiment, the fluid dispersal system comprises a common housing having a jacket that envelops the diffuser means, the accommodation means and the fluid container. The housing in particular comprises a diffusion outlet adapted to discharge a dispersion into the surrounding air or to introduce a dispersion to an air conditioning or ventilation system or a fan. In particular, the jacket is basically shaped cubical or cylindrical.

In one embodiment of this system the fluid container comprises a machine-readable code for allowing identification of the fluid container and/or of a fluid contained by the fluid container, wherein the machine-readable code is provided as a printed code, particularly an ultraviolet barcode, or as an RFID tag, and the housing comprises sensor means for reading the code, particularly wherein a camera or a barcode scanner is provided at the jacket and adapted to capture a printed code, particularly an ultraviolet barcode, of the fluid container, or an RFID reader is provided that is adapted to read an RFID tag of the fluid container.

In another embodiment of the fluid dispersal system according to this aspect of the invention, the feeder tube coupling comprises a male probe and the feeder tube comprises a female receiver that are adapted to create a fluid tight connection between the feeder tube coupling and the feeder tube.

In yet another embodiment of the fluid dispersal system according to this aspect of the invention, the accommodation means comprises an opening means that is adapted to open the seal when the fluid container is mounted to the accommodation means.

In one embodiment the opening means is a circular blade, particularly being adapted to cut the seal when the fluid container is mounted to the accommodation means before the feeder tube coupling couples with the feeder tube.

In another embodiment of the refillable fluid dispersal system, the system is electrically powered. In particular, a compressor of the system is powered by means of AC mains power, and the system comprises a power supply cord or means for connecting a power supply cord, the diffuser means is powered by means of an accumulator battery, and the system comprises means for connecting a power supply cord to recharge the accumulator battery, or the system comprises photovoltaic cells adapted to supply power to the diffuser means and/or to an accumulator battery.

A fluid container is adapted to contain a fluid and for providing the fluid to a fluid dispersal system according to the invention. The fluid container comprises a body with a corpus part enclosing a volume for accommodating the fluid, and a neck part having a first screw thread for connecting the fluid container to the fluid dispersal system. According to the invention, a feeder tube extends from the neck into the volume, wherein a centre point of a circular diameter of the feeder tube correlates with a centre point of a circular diameter of the screw thread, and a seal is provided at the outer end of the neck part to hermetically seal the fluid container and to prevent the fluid from exiting the fluid container.

In one embodiment, the fluid container comprises an insert wherein the feeder tube is part of the insert and wherein the insert comprises positioning means that are adapted to fixedly positioning the feeder tube in the fluid container.

In one embodiment of this fluid container, the positioning means are adapted to be installed and permanently affixed in the neck part of the fluid container by means of bonding or gluing. In another embodiment, the seal is a part of the insert.

In another embodiment, the fluid container comprises a machine-readable code for allowing identification of the fluid container and/or of a fluid contained by the fluid container, wherein the machine-readable code is provided as a printed code on the outside of the body, particularly an ultraviolet barcode, or as an RFID tag.

In yet another embodiment, the fluid container is a basically cylindrical screw-threaded vessel, wherein the diameter of the neck part corresponds to the diameter of the corpus part, or the diameter of the corpus part exceeds the diameter of the neck part, and the body is made from glass, PTE, plastic, aluminium, steel and/or other metal.

In another embodiment of the fluid container according to this aspect of the invention, the feeder tube is basically formed as a hollow cylinder and/or comprises a flexible tube, and made of glass, plastic, rubber, aluminium and/or other metal.

In a further embodiment of the fluid container according to this aspect of the invention, the seal is a liquid-, gas- and vapour-proof membrane, particularly wherein the seal is made from plastic and/or aluminium.

In one embodiment, the volume of the fluid container is at least partially filled with the fluid, particularly wherein the fluid comprises one or more essential oils, and/or is a water based solution, comprising air-dispersable fragrant, odour eliminator and/or disinfectant solutes.

An insert for use with a fluid container according to this aspect of aspect of the invention comprises a feeder tube, comprises positioning means that are adapted to fixedly positioning the insert in the fluid container so that the feeder tube extends from a neck part of the fluid container into a fluid-containing volume of the fluid container, and positioning the feeder tube so that a centre point of a circular diameter of the feeder tube correlates with a centre point of a circular diameter of the neck part.

In one embodiment, the insert comprises a seal that is adapted to hermetically seal the fluid container at the outer end of the neck part and to prevent the fluid from exiting the fluid container, particularly wherein the seal is a liquid-, gas- and vapour-proof membrane, particularly made from plastic and/or aluminium.

In another embodiment, the insert is adapted to be installed and permanently affixed in the fluid container, particularly by means of bonding or gluing a ring-shaped surface of the positioning means to the neck part of the fluid container, particularly wherein the ring-shaped surface is adaptable to the neck part with respect to the diameter.

In yet another embodiment of the insert according to this aspect of the invention, the feeder tube is basically formed as a hollow cylinder, comprises a flexible tube, is made of glass, plastic, rubber, aluminium and/or other metal, and/or is adapted to create a fluid tight connection with a feeder tube coupling of a fluid dispersal system according to the first aspect of the invention, particularly wherein the feeder tube comprises a female receiver for accepting a male probe of the feeder tube coupling.

In a further embodiment of the insert, the positioning means comprises a ring-shaped surface for contacting the inner surface of the neck part of the fluid container, particularly forming a fluid tight connection with the neck part, comprises at least one crossbeam to hold the feeder tube, and/or is made of glass, plastic, aluminium and/or other metal.

The invention in the following will be described in detail by referring to exemplary embodiments that are accompanied by figures, in which:
- Fig. 1: shows a refillable fluid dispersal system having prior art features;
- Figs. 2a-b: show a first exemplary embodiment of an automated refillable fluid dispersal system according to the invention;
- Figs. 3a-b: show a second exemplary embodiment of a fluid dispersal system according to the invention;
- Fig. 4: illustrates an exemplary operating mode of the cold air diffusion chamber.
- Fig. 5: is a table showing minimum and desired ratios of certain orifices of embodiments of a fluid dispersal system according to the invention;
- Fig. 6: shows a third exemplary embodiment of an automated refillable fluid dispersal system according to the invention;
- Figs. 7a-b: show an exemplary embodiment of a liquid level indicator system;
- Fig. 8: shows the housing of an exemplary embodiment of a fluid dispersal system in a sectional view;
- Fig. 9: shows a fluid container of an exemplary embodiment of a fluid disperser system in a sectional view;
- Figs. 10a-b: show an exemplary embodiment of a fluid container in a perspective drawing;
- Figs. 11a-b: show two exemplary embodiments of an insert in a perspective drawing;
- Figs. 12a-b: show an exemplary embodiment of the fluid dispersal system of Fig. 8 with and without a fluid container inserted;
- Figs. 13a-b: show the fluid container of Fig. 9 in a top view, with and without a sealing;
- Fig. 14: shows a coupling portion of the housing to accept the fluid container;
- Figs. 15a-c: show the insertion of a fluid container into a first embodiment of the housing; and
- Figs. 16a-c: show the insertion of a fluid container into an alternative embodiment of the housing.

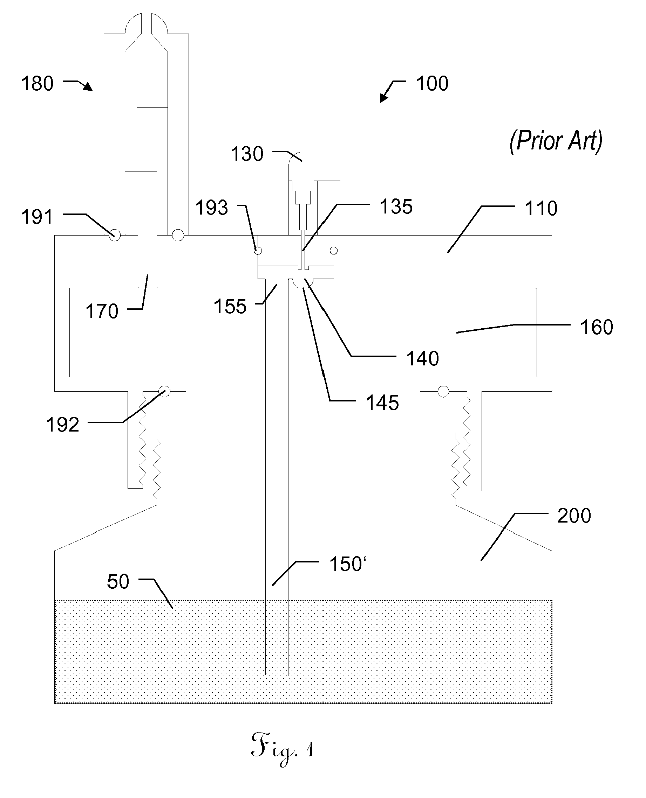

Figure 1 shows a typical prior art setup of an automated refillable fluid dispersal system using cold air diffusion. It comprises a diffuser head 110 to which an exchangeable fluid container is connected, e. g. by means of a screwed connection.

The diffuser head 110 comprises an air intake 130 where compressed air is drawn into the cold air diffusion chamber 140. As shown here, typically the air intake 130 narrows significantly from the entry point to an air jet nozzle 135 to increase the speed of airflow.

A feeder tube 150' is attached to the diffuser head 110 extending into the fluid container 200 in order to allow fluid 50 to be drawn to a fluid import 155 and into the cold air diffusion chamber 140.

Through the mix exhaust port 145 an air and fluid mix is drawn from the cold air diffusion chamber 140 into a mix chamber 160. From the mix chamber 160 fragrance vapours exit the device at speed and typically with a pressure of 2.7 bar (39 psi). Due to the pressure regime required the exiting vapour is often wet, hence a drying chamber 180 is required.

Seals and gaskets 191, 192, 193 may deform, double in size or decompose when in contact with commonly used fluids for dispersion, such as pure essential fragrance oil.

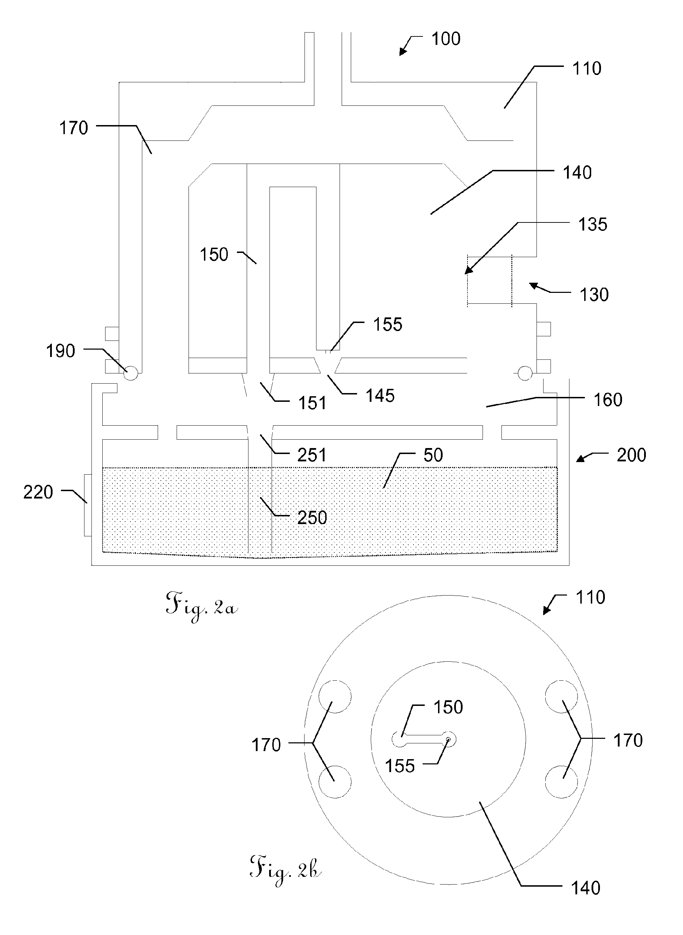

Figures 2a and 2b show a first embodiment of an automated refillable fluid dispersal system 100 according to the invention. Applicant has discovered that ratios between some of the various orifices are critical to the performance of the device and that by reversing current thinking on orifice size, pressure and air flow significant performance improvements can be achieved. Through the redesign of the atomising device, it is possible to significantly further increase the volume of dry vapour matter which can be created within such a device. When both discoveries are implemented together it is possible to greatly increase the output of a significantly dry vapour, particularly where the output is greater than 50 ml per hour of operation.

The depicted fluid dispersal system 100 of Figure 2a comprises a diffuser head 110 and an exchangeable fluid container 200 which is attachable thereto. In contrast to the prior art system of Figure 1, the feeder tube 250 is part of the container 200, the diffuser head 110 only having a feeder tube extension 150, and the feeder tube 250 and the feeder tube extension 150 being connectable by a first and second coupling 151, 251, e. g. a male probe and a female receiver, adapted to create a fluid tight connection.

The diffuser head 110 comprises an air intake 130 which in comparison to the prior art system of Figure 1 is relatively larger, narrowing from the entry point to an air jet nozzle 135 - if at all - only insignificantly.

The cold air diffusion chamber 140 is considerably larger, the fluid import 155 having an orifice that is relatively small.

Preferably, the mix exhaust port 145 is positioned opposite the fluid import 155 and the fluid import 155 is designed as a Venturi tube, so that the fluid 50 is drawn through the feeder tube 250, the feeder tube extension 150 and the fluid import 155 into the cold air diffusion chamber (Venturi chamber) 140 by means of the Venturi effect.

The mix chamber 160 can be basically formed by the upper portion of the fluid container 200. The mist output vents 170 are relatively large, so that the output dispersion can exit the device with considerably lower pressure and significantly dryer than with the system of Figure 1.

The O-Ring 190 is preferably made of an elastomer which is resistant to the essential or synthetic oils that can be dispersed by the system.

Figure 2b is a sectional view of the diffuser head 110 showing the cold air diffusion chamber 140 with the fluid import 155 and the feeder tube extension 150 connecting the fluid import 155 to the fluid container (not shown here). Four mist output vents 170 are arranged around the cold air diffusion chamber 140, together providing a large output vent orifice.

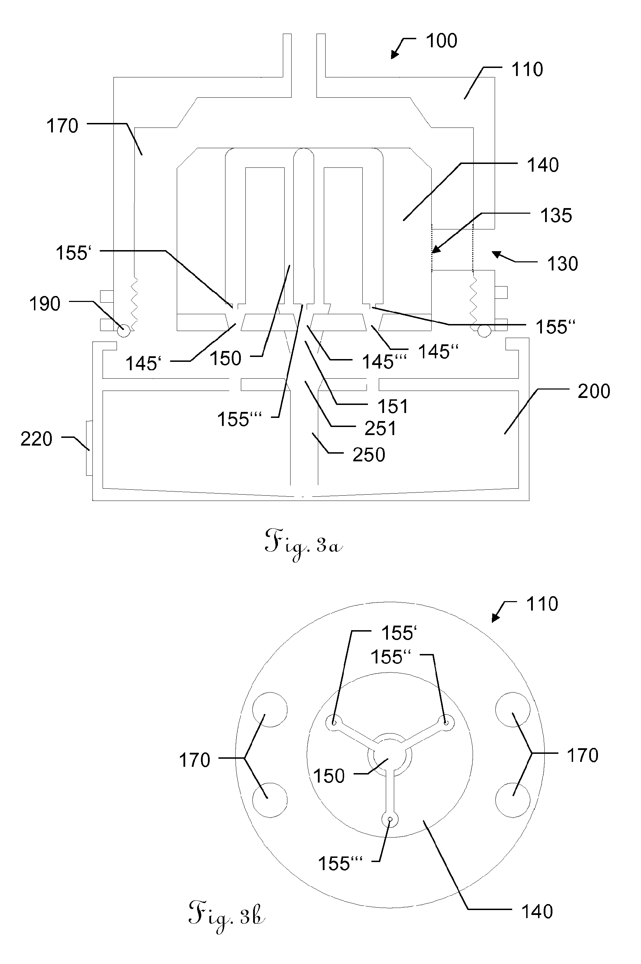

Figures 3a and 3b show a second embodiment of a fluid dispersal system 100 according to the invention.

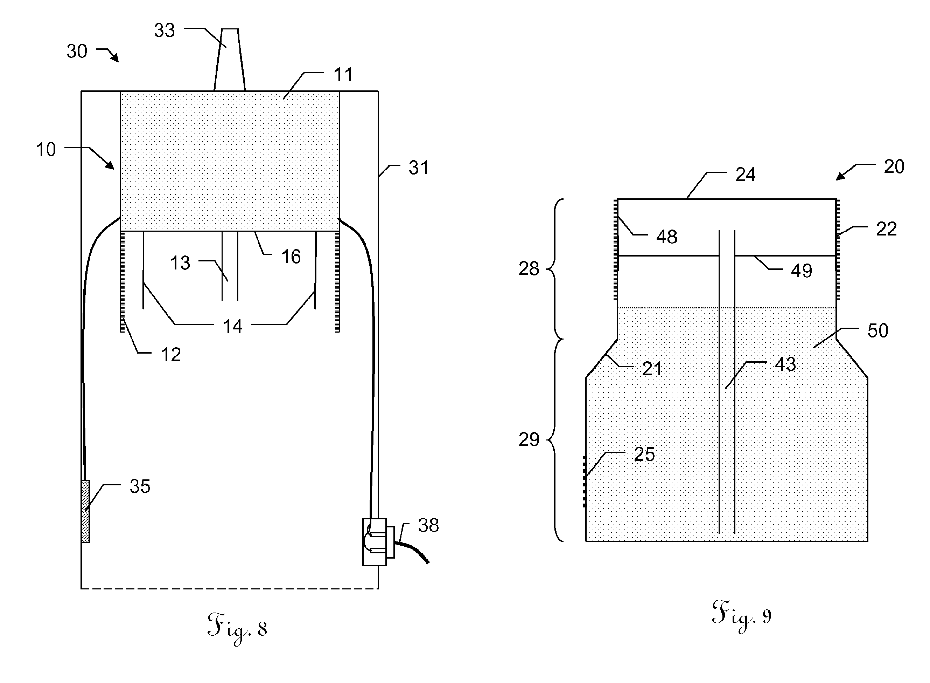

The depicted system differs from the first embodiment of Figures 2a-b firstly in that the feeder tube 250 is arranged centrally in the fluid container 200. The extension 150 is likewise arranged centrally in the diffuser head 110. As the first and second feeder tube couplings 151, 152 are thus arranged centrally, the fluid container 200 and the diffuser head 110 may be adapted to be connected by means of screwing. Such a screwed connection is further described with respect to Figures 8 to 16.

The depicted system differs from the first embodiment further in that - instead of a single fluid import - three fluid import units 155', 155", 155"' are provided, and the feed tube 250, and feed tube extension 150 have an increased diameter. This allows for a greater amount of fluid to be converted to dry mist output at a time.

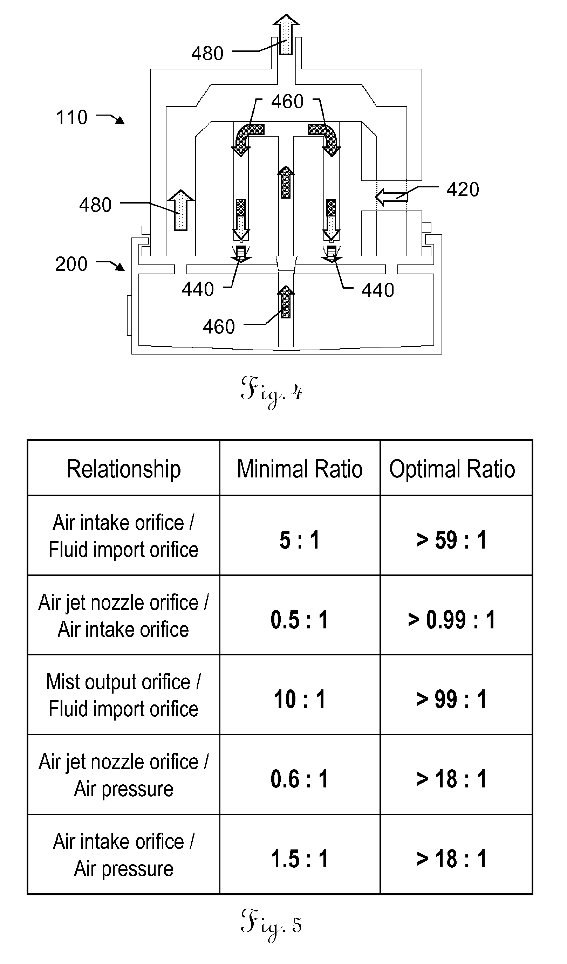

Figure 4 illustrates the flow of air 420, fluid 460 and dispersion 480 in the fluid dispersal system of Figure 3a. In Figure 4, the container 200 has been mounted to the diffuser means 110. Air 420 is injected into the diffusion chamber from the outside through the air intake with relatively low pressure, e. g. with a relative pressure of below 0.3 bar. The air is compressed and then exhausted as a high pressure air blast 440 through the diffusion chamber's exhaust ports. The exhaust ports and the fluid imports are arranged and adapted so that in the feeder tube low pressure (partial vacuum) is generated through the air blast 440. Consequently, the fluid 460 in the container 200 is sucked up through the feeder tube towards the fluid imports. The fluid imports are designed so as to disperse (e. g. aerolize or vaporize) the fluid (e. g. comprising a spray valve or a jet nozzle) and release it as dispersion 480 (e. g. mist or vapour) into the diffusion chamber and into the container 200. The pressure in the fluid import is significantly higher than the pressure at the air intake, e. g. at least 50% higher. According to the intended use of the device, the dispersion 480 is then dispersed out of the device via the dispersion output vents and through the outlet into the surrounding air - or alternatively introduced to a fan or an air conditioning or ventilation system from which the dispersion 480 is then dispersed into the air.

In the table of Figure 5 a number of relationships between orifice sizes and either other orifice sizes or air pressures is listed, together with minimum ratios and optimum ratios for improving the performance of the system.

Research by the applicant has shown that the ratios between certain orifices are critical to the performance of cold air diffusion systems and their ability to output dry particulate matter. The applicant has also discovered that by reversing the current thinking on orifice sizes and altering the ratios correspondingly, a significant performance improvement (up to 800%) can be achieved. This applies particularly in combination with a significant reduction of the air pressure.

In the first row, a relationship between a size of the orifice of the air intake and a size of the orifice of the fluid import is shown. Whereas in typical devices of the prior art this ratio is between 2.1:1 and 3.2:1, applicant has discovered that altering the ratio to at least 5:1 (having a relatively bigger air intake / smaller fluid import) already leads to a considerable increase of output mist. The output raises further together with this ratio, so that a ratio of about 30:1 the output is already significantly higher than what can be achieved in devices of the prior art. An optimized output can be achieved at a ratio of 59:1 or more.

In the second row, a relationship between a size of the orifice of the air jet nozzle and a size of the orifice of the air intake is shown. In typical devices of the prior art the nozzle is very small compared to the air intake, the ratio typically being between 0.25:1 and 0.43:1. The applicant has discovered that altering this ratio to at least 0.5:1 - i.e. making the diameter of the nozzle at least half as big as the diameter of the air intake, thus reducing the air pressure - leads to a significantly reduced wetness of the dispersed mist. The optimum ratio lies between 0.99:1 and 1:1.

In the third row, a relationship between a size of the mist output orifice and a size of the orifice of the fluid import is shown. Typical devices of the prior art use relatively small output orifices and relatively large fluid import orifices - the ratio being between about 4:1 to 6.3:1. Applicant has discovered that altering this ratio to at least 10:1, preferably at least 99:1, significantly increases the possible output thereby also significantly reducing the wetness of the output.

In the fourth and fifth rows, a relationship between on the one hand a size of the air jet nozzle orifice or a size of the air intake orifice (in mm²) and on the other hand an air pressure (in bar) is shown. For the air jet nozzle orifice the ratio is typically between 0.25:1 and 0.47:1, and for the air intake between 1.05:1 and 1.1:1. Applicant has discovered that altering these ratios to at least 0.6:1 and 1.8:1, preferably to at least 18:1, leads to a significant increase of output mist and decrease of mist wetness.

Of course, these ratios may be combined in order to achieve the best results.

This discovery allows for a system to output more scent fragrance while operating under lower pressure and for less time. Advantageously, this makes it suited to DC battery power - which is desirable in many locations where an AC power supply is difficult or expensive to provide.

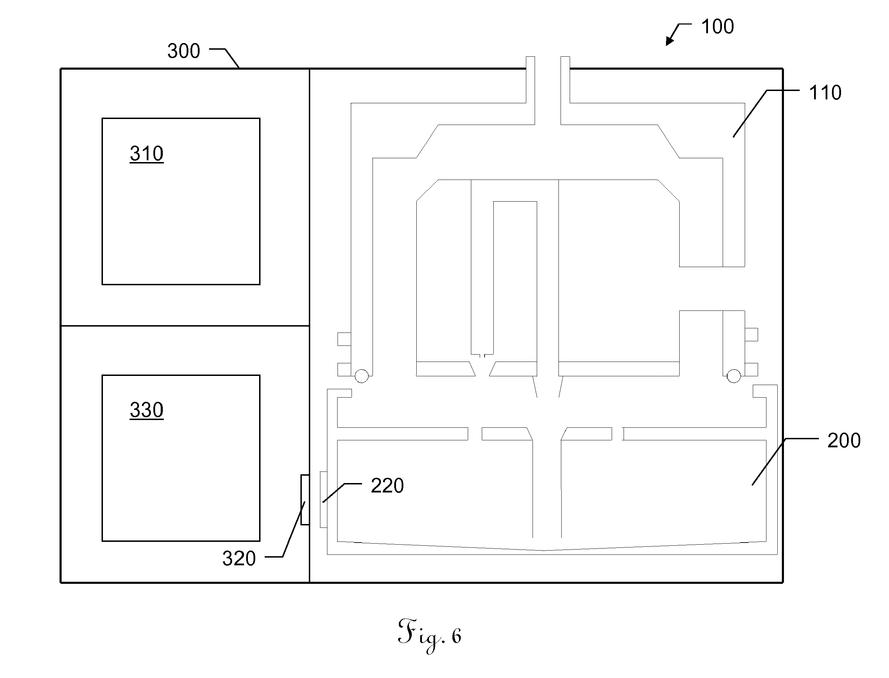

Figure 6 shows a third embodiment of a fluid dispersal system 100 according to the invention. In this embodiment, the diffuser means 110, the exchangeable liquid container 200 and further components are provided within a common housing 300.

Said further components comprise a sensor 320 for reading a code provided on the exchangeable liquid container 200, the sensor e. g. being an RFID sensor, a camera or a barcode scanner. Other components are e. g. a timer and programming module 310 and at least one air compressor 330.

There are many applications and business sectors for such a system, these include but are not limited to:
- Fragrancing: dispersing pure essential or synthetic oils into the air;
- Pathogen prevention and control: dispersing disinfectants into the air, onto surfaces, products and also onto hands;
- Pest control and prevention: dispersing pesticides into the air, onto surfaces, products and livestock within food production and animal husbandry;
- Agriculture / food production: dispersing nutrients and shelf life extending chemicals onto plants and food products;
- Restoration: dispersing chemicals onto wooden surfaces for the purposes of combatting and treating wood weakening life forms;
- Security: dispersing chemicals onto products and people for the purposes of identification and tracking.

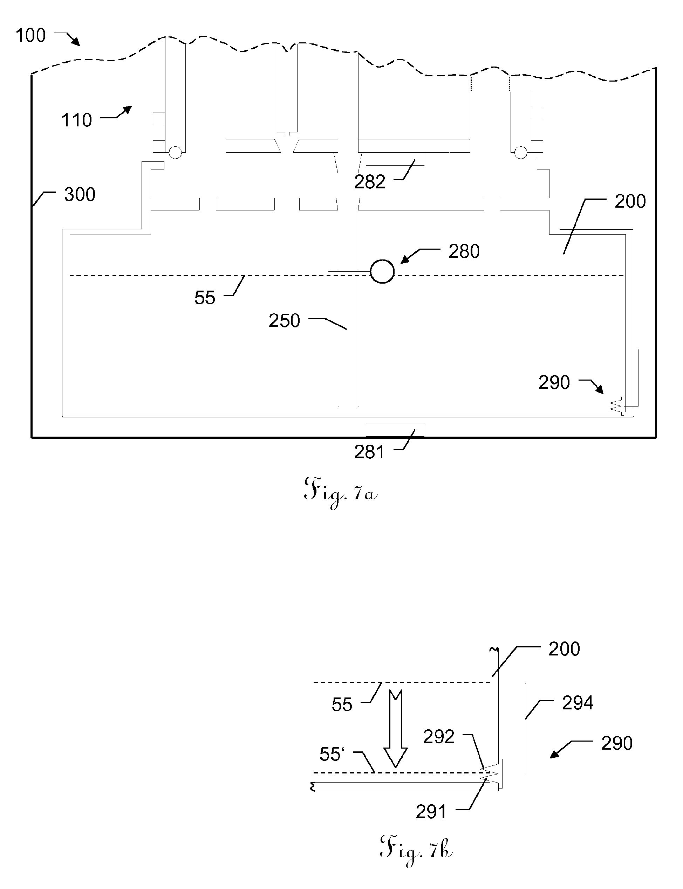

Figures 7a and 7b relate to another aspect of the invention referring to liquid level indicator systems.

Figure 7a shows a liquid container 200 within the fluid dispersal system 100 of Figure 6 having a fluid level 55. Together with the system 100 two different, independently working liquid level indicator systems are depicted. This is mainly for presentation reasons, as - although it would be possible to use both systems together - the systems are basically redundant.

The first liquid level indicator comprises a float 280 inside the liquid container 200 that is connected to the container's feeder tube 250 by means of a ring and adapted to float on the fluid thus indicating a current level 55 of the fluid inside the liquid container 200. The float comprises an RFID transponder 280, or a similar transponder means. The system further comprises at least one RFID sensor 281, 282 (or other suitable sensor means to detect the transponder). This is provided above or below the liquid container 200.

These RFID sensors 281, 282 use very low power RFID to facilitate this desired functionality where the transponder 280 will float on the fluid within the container 200, only becoming detectable by a sensor 281, 282 if it gets extremely close to this.

If the detection element is situated below the refill, a detection of the RFID transponder 280 by sensor 281 can therefore be interpreted as a low fluid level 55. If the detection element is situated above the refill, a detection of the RFID transponder 280 by sensor 282 can likewise be interpreted as a high fluid level 55. If both sensors 281, 282 are provided, and neither of the two sensors 281, 282 detect the transponder 280, this can be interpreted as a medium fluid level 55. The determined fluid level 55 can be signalled to a user of the device, e. g. by coloured LED: green for a high level, yellow for a medium level and red for a low level. A determined low fluid level can also cause a signal to replace the container 200.

The RFID sensors preferably are rather weak, and thus only able to identify the transponder through the container body if it is a few millimetres to some centimetres away (depending on the size of the container). For instance, the RFID sensors can be adapted to identify the transponder in a maximum range of five millimetres or of two centimetres.

The second liquid level indicator comprises a capacitance sensor 290 and is suitable for conductive fluids. The capacitance sensor 290 is depicted in greater detail in Figure 7b. It comprises two capacitance probes 291, 292 that are punctured through the side of the fluid container 200 at low level and connected by means of a cable 294 to a processor of the system 100 to detect when it is time to replace an empty fluid container 200. If the level 55 of the conductive fluid is high enough an electrical connection across both probes is enabled. As the level 55' drops below the height of the upper probe 292, the connection is broken, which is interpreted as a signal to replace the container 200.

With either liquid level indicator, a signal to replace the fluid container 200 can be given to the user, e. g. by means of a sound or light signal, to the device, which e. g. stops operating, and also to a supplier of the fluid container 200, e. g. by means of an Internet connection of the device. The supplier can then provide the necessary refill.

Although the liquid level indicator systems are shown as a part of a fluid dispersal system, it may be used for other systems as well.

The Figures 8 to 16 relate to a further aspect of the invention referring to a design of a fluid dispersal system wherein the fluid container comprises the feeder tube. In the depicted system the container is attachable to the diffuser means by a screwed connection; it can however as well be attachable by other means, such as a compression fitting or a bayonet mount.

Figure 8 shows an electrical powered diffuser means 10 of an exemplary embodiment of the fluid dispersal system according to the invention in a sectional view. The diffuser means 10 is provided inside of a housing 30 that is adapted to also envelop a fluid container when connected to the diffuser means 10. The depicted diffuser means 10 comprises a cold air diffusion chamber 11 in which fluids can be vaporized and from which the vapour is dispersed.

Below the diffusion chamber 11, an accommodation means is provided comprising a screw thread 12 that is adapted to accept a screw thread of a fluid container so that the fluid container is screwable onto the accommodation means. A bottom face 16 of the accommodation means is adapted to evenly contact a top face of the fluid container, particularly in order to create a fluid tight connection with the fluid container. The accommodation means further comprise a feeder tube coupling 13 which connects as a male part with a feeder tube of the fluid container as a female part in order to create a fluid tight connection between the feeder tube coupling 13 and the feeder tube. Furthermore, in this embodiment a circular blade 14 is provided as an opening means. The blade is adapted to cut a sealing of the fluid container when the fluid container is screwed onto the accommodation means and before the feeder tube coupling 13 couples with the feeder tube.

The depicted housing 30 comprises a jacket 31, particularly having a cubical or cylindrical shape allowing the housing to stand on an even surface. The housing 30 comprises an outlet 33 through which the vapour from the diffusion chamber 11 is dispersed.

As in this embodiment a compressor of the system (not shown) is powered by means of alternating currents (AC) mains power, the housing comprises a plug-in connection for connecting a power supply cord 38.

The housing 30 comprises sensor means 35 for reading a machine-readable code on the fluid container, the code allowing the system to identify the fluid container, or the fluid inside the container, respectively. In this example, the sensor means 35 are embodied as a camera or a barcode scanner that is provided on the inside of the jacket 31 and adapted to capture a printed code on the fluid container, preferably, the code can be provided as an ultraviolet barcode. Alternatively, other sensors can be used, e. g. an RFID reader can be provided that is adapted to read an RFID tag of the fluid container. This sensor means 35 interfaces with a control timer printed circuit board and processor system of the diffuser means 10 and it will not permit the machine to operate until the sensor means 35 detects an original code that is within pre-programmed parameters, nor will it accept any code that it has previously been recognised and approved.

Figure 9 shows a fluid container 20 of an exemplary embodiment of a fluid dispersal system according to the invention in a sectional view, the fluid container 20 being adapted to be used with the diffuser means 10 of Figure 1. The fluid container 20 has a body 21 made from glass, plastic, aluminium or steel and comprises a neck part 28 and a corpus part 29. The neck part 28 comprises a screw thread 22 that is adapted fit on the screw thread of the accommodation means so that the fluid container is screwable onto the accommodation means, and the corpus part 29 encloses a volume that accommodates the fluid 50.

For instance, the fluid 50 can comprise one or more essential oils or be a water based solution. It can comprise air-dispersable fragrant, odour eliminator or disinfectant solutes or a combination thereof. Alternatively, the fluid 50 can comprise insect repellents or insecticides, e. g. for repelling or killing mosquitoes or bed bugs.

The fluid container 20 comprises an insert having a feeder tube 43 and positioning means 49 adapted to fixedly position the feeder tube 43 in the fluid container 20. The feeder tube 43 is adapted to connect with the feeder tube coupling of Figure 8. The fluid container 20 also comprises a seal 24 that prevents the fluid 50 from leaving the container 20. It can be provided as a part of the insert or separately. The insert is connected to the body 21 of the container by means of a ring-shaped surface 48 which contacts the inner surface of the neck part 28, preferably forming a liquid-tight connection with the neck part 28. The insert can be permanently fixed to the body, e. g. by means of bonding or gluing.

On the body 21, a machine-readable code is provided that allows identification of the fluid container 20 or its fluid, as described above. The amount of fluid that is vaporized can be controlled according to the kind of identified fluid. In this case, the code is an ultraviolet barcode 25 that is invisible to the human eye and positioned in such a way that the sensor of Figure 8 is able to read the encoded information. Preferably, the same barcode 25 is printed on the body 21 at more than one position. Alternatively, the machine-readable code can be provided by an RFID tag.

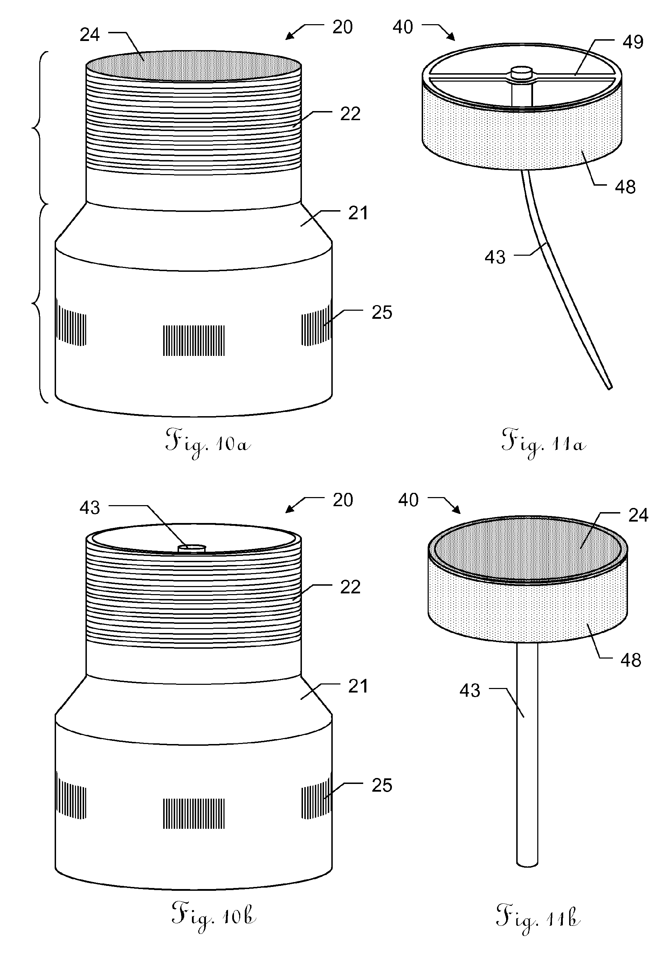

In Figures 10a and 10b the fluid container 20 of Figure 2 is shown in a front view. In Figure 10a, the seal 24 on top of the container 20 is present and unbroken; in Figure 10b, the seal has been removed in order to show the top part of the feeder tube 43 beneath the seal 24. As already described with respect to Figure 2, a multitude of identical barcodes 25 are provided on the body 21 at the same height.

Figures 11a and 11b show two embodiments of an insert 40. The inserts 40 are to be inserted into the fluid container of Figure 2 and then fixed inside. Both inserts 40 comprise a feeder tube 43 and positioning means 49 that are adapted to position the feeder tube 43 centrally in the neck part of the fluid container in such a way that at the top of the feeder tube 43 a centre point of a circular diameter of the feeder tube 43 correlates with a centre point of a circular diameter of the neck part, and so that the feeder tube 43 extends from the neck part into a fluid-containing volume of the fluid container to be submerged into the fluid. The positioning means 49 also comprise a ring-shaped surface 48 that is adapted to contact the inside surface of the container's neck part and create a liquid-tight connection with the inside surface. The ring-shaped surface 48 can also be fixedly connected to the inside surface by means of bonding or gluing.

In Figure 11a the feeder tube 43 comprises a flexible tube, e. g. made of rubber or plastic, whereas in Figure 11b, it is basically formed as a hollow cylinder, e. g. made of glass, plastic, aluminium or other metal. Additionally, the embodiment of Figure 11b differs from that of Figure 11a in that the sealing 24 is provided as a part of the insert 40.

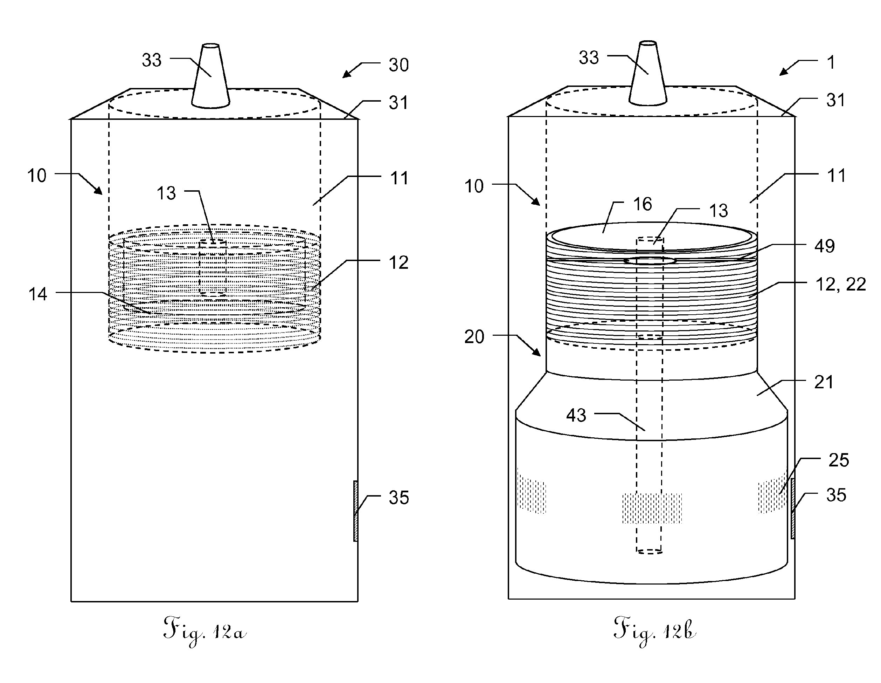

Figures 12a and 12b show the fluid dispersal system 1 in a cutaway view, wherein Figure 12a shows the system 1 with no fluid container 20 inserted.

In Figure 12b, a fluid container 20 has been connected to the diffuser means 10 by means of screwing the screw thread 22 of the container into the screw thread 22 of the device. In this position, the container 20 contacts the bottom face 16, particularly hermetically sealing the content of the container from the environment of the system 1.

A circular blade 14 (visible in Figure 12a) has cut away the seal of the container 20 before the feeder tube connector 13 has formed a fluid tight connection with the feeder tube 43 of the container 20. The feeder tube 43 is held in place in the container 20 by positioning means 49. A number of UV barcodes 25 is printed on the outer surface of the container's body 21, one of which being positioned to be readable by a barcode reader device 35, e. g. a miniature barcode camera.

Preferably, the neck portion of the fluid container 20 and the accommodation means are adapted to allow the following sequence of steps, when the fluid container 20 is screwed to the dispenser means 11:
1. establishing a fluid tight connection between the two screw threads 12,22;
2. opening the seal 24 of the container by the opening means 14;
3. connecting the feeder tube 43 and the feeder tube connector 13; and
4. contacting the bottom face 16 of the accommodation means with the top face of the container 20.

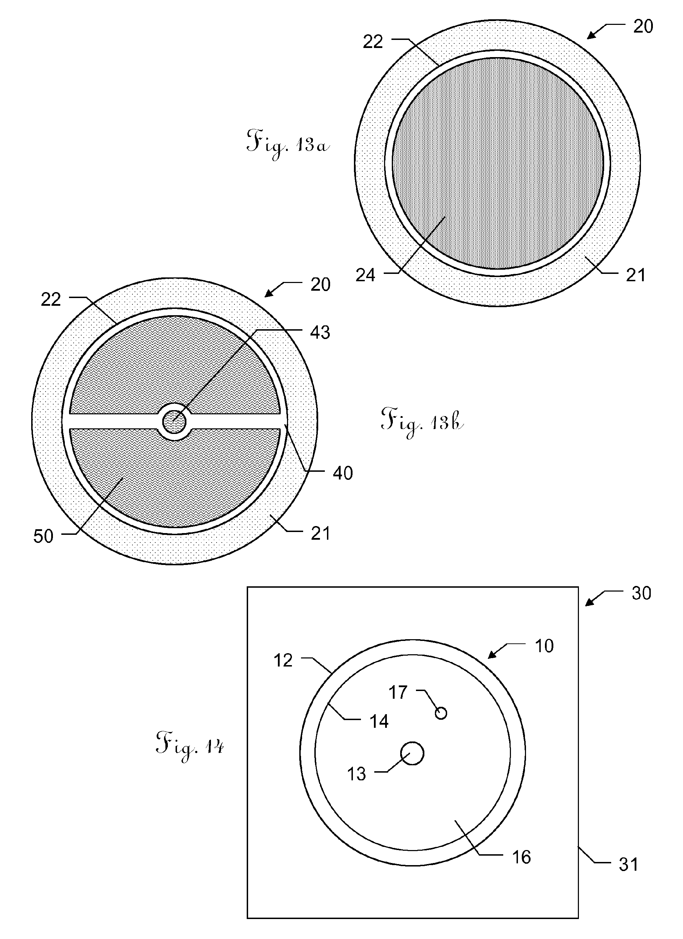

Figures 13a and 13b show the fluid container 20 of Figure 2 in a top view. The screw thread 22 on the outside of the neck part can be seen as well as the body 21 of the corpus part, as the diameter of the corpus part exceeds that of the neck part. In Figure 13a, the seal 24 is visible.

In Figure 13b, the seal is removed in order to allow the view inside of the container 20. The insert 40 is visible with the positioning means 49 holding the feeder tube 43 centrally in the container 20. Below the positioning means 49, the fluid 50 is shown.

Figure 14 shows the accommodation section of the diffuser means 10 in a top view. This section is adapted to accept the top part of the fluid container 20 shown in Figures 5a-b. It is enveloped by the cubical jacket 31 of the housing 30 and comprises a screw thread 12 into which the screw thread of the container is to be screwed. The screw thread 12 encompasses the bottom face 16 from which the circular blade 14 and the feeder tube coupling 13 protrude. The accommodation section further comprises an exhaust port 17 of the cold air diffusion chamber, through which compressed air is exhausted as a high pressure air blast.

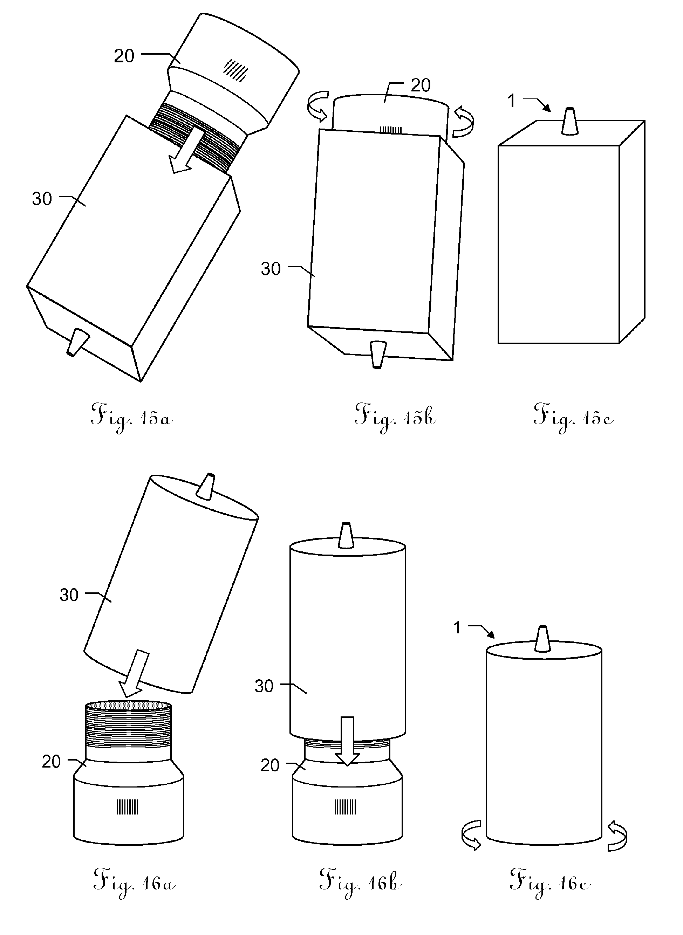

In Figures 15a-c and 16a-c, the insertion of the fluid container 20 into the housing 30 and the connection to form a system 1 according to the invention is illustrated using two different embodiments of the housing 30 are illustrated. If the accommodation means comprise opening means to breach the seal - such as a circular blade - the container 20 is sealed and can be inserted upside down to facilitate the connection with the diffuser means. The two screw threads are adapted to form a fluid tight connection before the integrated opening means of the system 1 open the seal of the container 20.

Although the invention is illustrated above, partly with reference to some preferred embodiments, it must be understood that numerous modifications and combinations of different features of the embodiments can be made. In particular, the three presented aspects of the invention can be combined with another in multiple ways. All of these modifications and combinations lie within the scope of the appended claims.

## Claims

1. Fluid dispersal system (100) comprising a diffuser means (110) with a diffusion chamber (140) adapted to disperse a fluid (50) by means of cold air diffusion, wherein the diffuser means (110) comprises
- an air intake (130) having an air intake orifice through which compressed air (420) is introducible into the diffusion chamber (140),
- a fluid import (155) having a fluid import orifice through which the fluid (50) is introducible into the diffusion chamber (140),
- a mix exhaust port (145) through which an air and fluid mix (440) from the diffusion chamber (140) is introducible into a mix chamber, and
- a mist output vent (170) that is connected to the mix chamber and adapted to allow a dispersion (480) from the mix chamber to be dispersed out of the system (100),
**characterized in that**
a ratio of cross-sectional areas of the air intake orifice to the fluid import orifice is at least 5:1.

2. Fluid dispersal system (100) according to claim 1,
**characterized in that**
the ratio of cross-sectional areas of the air intake orifice to the fluid import orifice is at least 30:1, in particular at least 59:1.

3. Fluid dispersal system (100) according to claim 1 or claim 2,
**characterized in that**
- the air intake (130) comprises an air jet nozzle (135) having a jet nozzle orifice; and
- the ratio of cross-sectional areas of the jet nozzle orifice to the air intake orifice is at least 0.5:1, particularly at least 0.99:1.

4. Fluid dispersal system (100) according to any one of the preceding claims,
**characterized in that**
- the mist output vent (170) has an output orifice; and
- the ratio of cross-sectional areas of the output orifice to the fluid import orifice is at least 10:1, particularly at least 99:1.

5. Fluid dispersal system (100) according to any one of the preceding claims,
**characterized in that**
the fluid import (155) comprises a Venturi tube, and the diffuser means (110) is adapted to draw the fluid (50) through the fluid import (155) into the diffusion chamber (140) by means of the Venturi effect.

6. Fluid dispersal system (100) according to any one of the preceding claims,
**characterized in that**
- in the diffusion chamber (140) the fluid import (155) is positioned oppositely the exhaust port (145);
- the fluid import (155) has an annular orifice, particularly wherein the first feeder tube coupling (151) is positioned in a centre of the annular orifice; and/or
- the fluid import comprises a multitude of fluid import units (155', 155", 155"'), particularly wherein a corresponding number of exhaust port units (145', 145", 145"') is provided, and in the diffusion chamber (140) each of the multitude of exhaust port units (145', 145", 145"') is positioned oppositely a fluid import unit (155', 155", 155"').

7. Fluid dispersal system (100) according to any one of the preceding claims,
**characterized in that**
- the compressed air (420) is introducible through the air intake (130) with a first air pressure, and the ratio of the air intake orifice size in mm² to the first air pressure in bar is at least 1.5:1, particularly at least 18:1, and/or
- the air intake (130) comprises an air jet nozzle (135) having a jet nozzle orifice size, the compressed air (420) is introducible through the air jet nozzle (135) with a second air pressure, and the ratio of the jet nozzle orifice size in mm² to the second air pressure in bar is at least 0.6:1, particularly at least 18:1.

8. Fluid dispersal system (100) according to any one of the preceding claims,
**characterized in that**
the output vent (170) is adapted to introduce the dispersion (480) to a fan or an air conditioning or ventilation system from which the dispersion (480) is dispersed.

9. Fluid dispersal system (100) according to any one of the preceding claims, the system comprising a fluid container (200) for providing the fluid (50),
**characterized by**
a common housing (300) for the diffuser means (110) and the fluid container (200), particularly wherein the system (100) further comprises
- a timer and programming module (310) adapted to control a dispersal programme of the diffuser means (110);
- at least one air compressor (330); and/or
- an accumulator battery and means for recharging the accumulator battery, particularly comprising photovoltaic cells or means for connecting a power supply cord.

10. Fluid dispersal system (100) according to any one of the preceding claims, the system comprising an exchangeable fluid container (200) for providing the fluid (50),
**characterized in that**
the diffuser means (110) comprises
- accommodation means adapted to accept the fluid container (200), and
- a feeder tube extension (150) with a first feeder tube coupling (151),
and is adapted
- to connect the fluid container (200) to the fluid import (155) so that the first feeder tube coupling (151) couples with a second feeder tube coupling (251) of a feeder tube (250) of the fluid container (200), and
- to draw the fluid (50) through the feeder tube (250) and the feeder tube extension (150).

11. Fluid dispersal system (100) according to claim 10,
**characterized in that**
- the first and second feeder tube couplings (151, 251) are adapted as a male probe and a female receiver and to create a fluid tight connection between the feeder tube extension (150) and the feeder tube (250); and/or
- the accommodation means is adapted to connect the fluid container (200) to the diffuser means (110) so that the mix chamber is created at least partially inside the fluid container (200).

12. Fluid dispersal system (100) according to claim 10 or claim 11,
**characterized in that**
- the accommodation means comprises a screw thread and is adapted to accept the fluid container (200) by means of screwing, and the feeder tube coupling is arranged at a centre point of the screw thread;
- the accommodation means is adapted to accept a plurality of exchangeable fluid containers (200) simultaneously, comprises a plurality of feeder tube extensions (150), and is adapted to connect the plurality of fluid containers (200) to the diffuser means (110) so that each of the feeder tube extensions (150) couples with a feeder tube (250) of one of the plurality of fluid containers (200); and/or
- the system comprises sensor means (320) for reading a machine-readable code (220) provided on the fluid container (200) and for identifying, based on the code (220), the fluid container (200) and/or a fluid (50) contained by the fluid container (200), particularly wherein the sensor means (320) comprises a camera, a barcode scanner or an RFID reader.

13. Exchangeable fluid container (200) that is adapted to contain a fluid (50) and for providing the fluid (50) to a diffuser means (110) of a fluid dispersal system (100) according to any one of the preceding claims when being connected to the diffuser means (110),
**characterized in that**
the fluid container (200) comprises a feeder tube (250) with a second feeder tube coupling (251) and is adapted to be connected to the diffuser means (110) in such a way that the second feeder tube coupling (251) couples with the first feeder tube coupling (151) of the diffuser means (110).

14. Method for dispersing a fluid (50) by means of cold air diffusion, particularly by means of a fluid dispersal system (100) according to any one of claims 1 to 12, the method comprising
- introducing air (420) with an air intake pressure through an air intake (130) into a diffusion chamber (140), and
- introducing the fluid with a fluid import pressure through a fluid import (155) into the diffusion chamber (140),
**characterized in that**
the fluid import pressure is significantly higher than the air intake pressure.

15. Method according to claim 14,
**characterized by**
- connecting a fluid container (200) to a diffuser means (110), wherein the fluid container (200) provides the fluid (50), the diffuser means (110) comprises the diffusion chamber (140), and connecting comprises coupling a feeder tube (250) of the fluid container (200) to a feeder tube extension (150) of the diffuser means (110); and/or
- drawing the fluid through the fluid import (155) by means of the Venturi effect, particularly wherein the air intake pressure is less than 0.3 bar.
